# EUROPEAN PATENT APPLICATION

(11) **EP 3 222 207 A1**
(43) Date of publication of application: **27.09.2017**
(21) Application number: 16305323.4
(22) Date of filing: 23.03.2016
(51) Int. Cl.: A61B 5/00, G06F 19/00, A61B 5/11, A61B 5/16

(54) **SYSTEM AND METHOD FOR NON-INTRUSIVE DETECTION AND MONITORING OF PARKINSON'S DISEASE SYMPTOMS**

(71) Applicant: Thomson Licensing, 92130 Issy-les-Moulineaux (FR)
(72) Inventor: LONGET, Aurélien, 35576 Cesson-Sévigné (FR); GUEGAN, Marie, 35576 Cesson-Sévigné (FR); LAMBERT, Anne, 35576 Cesson-Sévigné (FR)
(74) Representative: Huchet, Anne

(57) **Abstract**

A method and apparatus for creating an anomalous behavior detection log for reporting anomalous behavior of a monitored individual in an environment are described including training an anomaly detection classifier using first time-stamped event data for an initial period of time and raw sensor data, accepting second time-stamped event data of subsequent behavior from sensors in the environment for a subsequent period of time, logging the second time-stamped event data of subsequent behavior of the monitored individual, mining an action sequence based on a selected subset of mined manipulation patterns, calculating an anomaly score of each mined action sequence using the anomaly detection classifier and creating an anomalous behavior detection log based on the anomaly scores.

## Description

### FIELD

The proposed method and apparatus relates to the detection and monitoring of symptoms of Parkinson's disease in individuals living in restrictive environments.

### BACKGROUND

This section is intended to introduce the reader to various aspects of art, which may be related to the present embodiments that are described below. This discussion is believed to be helpful in providing the reader with background information to facilitate a better understanding of the various aspects of the present disclosure. Accordingly, it should be understood that these statements are to be read in this light.

Parkinson's disease is a neurological disorder that occurs when certain neurons in the brain die or become impaired. These nerve cells are located in a midbrain structure that controls muscle movement and produces dopamine, the chemical responsible for coordinated muscle function. Symptoms of Parkinson's disease occurs when 40 to 50 percent of nerve cells in the brain that produce the neurotransmitter dopamine have already died.

According to several studies, it is estimated that 6.3 million people have Parkinson's disease worldwide, affecting all races and cultures, with 4 to 20 new cases reported per 100,000 people per year. The condition usually affects those over age 65. Between 1% and 2% of senior citizens have some form of the disease world-wide.

Persistent body tremor is the most common symptom of Parkinson's disease in the elderly. Sluggish movement, stiffness and challenges with balance are also indicators, as are hand cramps, shuffling, frozen facial expressions, muffled speech patterns, and depression. Symptoms of Parkinson's patients progress and worsen over time. Increased tremors affect dexterity, while movement slows considerably (called bradykinesia). These physical changes impact the most routine habits like getting dressed or rising from a chair. Posture begins to stoop, as the head and shoulders press forward to compensate for the apparent lack of balance. Symptoms may occur on one or both sides of the body, but typically begin on one side and eventually spread to the other side as well.

Parkinson's disease in the elderly is not easily diagnosed, as neither x-rays nor blood tests reveal the condition, though blood tests and magnetic resonance imaging can be used to eliminate other conditions. Furthermore, there is so much variability among patients in how the disease progresses, doctors are not able to accurately predict how quickly symptoms will worsen, or even which specific symptoms will develop for each patient. This makes Parkinson's disease in the elderly a difficult disease to diagnose properly.

More background information on the disease and its attributes can be found in medical textbooks and journals. Another point to consider, as revealed by an April 2011 survey by the National Parkinson's Foundation, people will avoid visiting the doctor to discuss Parkinson's disease even when experiencing worrisome symptoms, such as tremors. The problem, however, is that waiting delays beginning treatment that, although it can't *cure* Parkinson's, can buy time.

Currently, the only system to monitor victims of Parkinson's disease in their homes are miniature sensors (often included in a device attached to the wrist, similar to a watch for example) attached to the body to capture movement characteristics associated with parkinsonian symptoms. Usually the monitored individual is in a restrictive environment such as senior housing. But such a device is very intrusive for the patient, and many elder persons do not want to have a sensor on their body all the time.

Another disadvantage is that the installation of such monitoring systems comes often when the disease is already advanced, and can be detected easily by the elder himself or by doctors in a routine control. So it is already too late.

In the prior art the capacity to evaluate Parkinson's disease tremors using wireless accelerometer technology has been proven possible using the built-in accelerometer of an iPhone, which is attached to the back of an elderly person's hand. However this is limited in scope as there is no interaction with the phone since it is attached to the back of the hand.

### SUMMARY

For better adoption of a monitoring system, the system needs to be non-intrusive, and to be totally transparent regarding elder habits. The proposed method and apparatus is non-intrusive system and detects and monitors early signs of Parkinson's symptoms and does not require any change of behavior from the elderly person.

The proposed method and apparatus uses sensors included in elder TV or STB remote control devices to detect slight shaking, tremors, and other Parkinson's disease symptoms that can appear during the execution of a specific pattern of remote control key manipulation. Coupled with specific algorithms, sensors capture recorded data during a specific remote control manipulation pattern. The data can be analyzed for a specific person or compared to scores generated by neurologists and used to measure the severity of Parkinson's disease symptoms.

An advantage is that almost every elderly person uses television every day, so they are also using the remote control device naturally several times every day. The elderly monitored individual does not have to change their habits. Data will be collected and analyzed transparently. Another advantage is that detection of early signs of Parkinson's disease is possible, even before elderly monitored person has noticed any symptoms. Thus, the proposed method and apparatus will be able to raise alerts to the monitored individual or their care giver to encourage the monitored individual to see his/her doctor. Such early detection can allow treatment to start very soon in the disease life, and maximize the chance of preserving the elderly individual's health. In routine care, using data recorded in the home environment can also facilitate adjusting medications to minimize fluctuation and encourage the monitored individual to continue treatment. Doctors and care givers can use the proposed method and apparatus to monitor already detected patients and help them in the analysis of disease evolution.

A method and apparatus for creating an anomalous behavior detection log for reporting anomalous behavior of a monitored individual in an environment are described including training an anomaly detection classifier using first time-stamped event data for an initial period of time and raw sensor data, accepting second time-stamped event data of subsequent behavior from sensors in the environment for a subsequent period of time, logging the second time-stamped event data of subsequent behavior of the monitored individual, mining an action sequence based on a selected subset of mined manipulation patterns, calculating an anomaly score of each mined action sequence using the anomaly detection classifier and creating an anomalous behavior detection log based on the anomaly scores.

### BRIEF DESCRIPTION OF THE DRAWINGS

The proposed method and apparatus is best understood from the following detailed description when read in conjunction with the accompanying drawings. The drawings include the following figures briefly described below:
Fig. 1 is an illustration of a remote shaking movement (tremor symptom) of Parkinson's disease.
Fig. 2 is an illustration of a remote oscillating movement (tremor symptom) of Parkinson's disease.
Fig. 3 is an illustration of a remote large movement (unwanted acceleration symptom) of Parkinson's disease.
Fig. 4 is a schematic diagram of an exemplary embodiment of the proposed apparatus.
Fig. 5 is an exemplary embodiment of the proposed method and apparatus.
Fig. 6 is a flowchart of an exemplary embodiment of the proposed method.
Fig. 7 is a flowchart of an exemplary embodiment of the training phase of the proposed method and apparatus.
Fig. 8 is a flowchart of an exemplary embodiment of the anomaly detection phase of the proposed method and apparatus.
Fig. 9 is a block diagram of an exemplary embodiment of the proposed method and apparatus.

It should be understood that the drawing(s) are for purposes of illustrating the concepts of the disclosure and is not necessarily the only possible configuration for illustrating the disclosure.

### DETAILED DESCRIPTION

The present description illustrates the principles of the present disclosure. It will thus be appreciated that those skilled in the art will be able to devise various arrangements that, although not explicitly described or shown herein, embody the principles of the disclosure and are included within its scope.

All examples and conditional language recited herein are intended for educational purposes to aid the reader in understanding the principles of the disclosure and the concepts contributed by the inventor to furthering the art, and are to be construed as being without limitation to such specifically recited examples and conditions.

Moreover, all statements herein reciting principles, aspects, and embodiments of the disclosure, as well as specific examples thereof, are intended to encompass both structural and functional equivalents thereof. Additionally, it is intended that such equivalents include both currently known equivalents as well as equivalents developed in the future, i.e., any elements developed that perform the same function, regardless of structure.

Thus, for example, it will be appreciated by those skilled in the art that the block diagrams presented herein represent conceptual views of illustrative circuitry embodying the principles of the disclosure. Similarly, it will be appreciated that any flow charts, flow diagrams, state transition diagrams, pseudocode, and the like represent various processes which may be substantially represented in computer readable media and so executed by a computer or processor, whether or not such computer or processor is explicitly shown.

The functions of the various elements shown in the figures may be provided through the use of dedicated hardware as well as hardware capable of executing software in association with appropriate software. When provided by a processor, the functions may be provided by a single dedicated processor, by a single shared processor, or by a plurality of individual processors, some of which may be shared. Moreover, explicit use of the term "processor" or "controller" should not be construed to refer exclusively to hardware capable of executing software, and may implicitly include, without limitation, digital signal processor (DSP) hardware, read only memory (ROM) for storing software, random access memory (RAM), and nonvolatile storage.

Other hardware, conventional and/or custom, may also be included. Similarly, any switches shown in the figures are conceptual only. Their function may be carried out through the operation of program logic, through dedicated logic, through the interaction of program control and dedicated logic, or even manually, the particular technique being selectable by the implementer as more specifically understood from the context.

In the claims hereof, any element expressed as a means for performing a specified function is intended to encompass any way of performing that function including, for example, a) a combination of circuit elements that performs that function or b) software in any form, including, therefore, firmware, microcode or the like, combined with appropriate circuitry for executing that software to perform the function. The disclosure as defined by such claims resides in the fact that the functionalities provided by the various recited means are combined and brought together in the manner which the claims call for. It is thus regarded that any means that can provide those functionalities are equivalent to those shown herein.

The proposed method and apparatus concentrates on the detection and analysis of three symptoms of Parkinson's disease:
- **Tremors:** Tremors include a shaking or oscillating movement, usually begins in a limb, often a hand or fingers.
- **Unwanted Accelerations:** It is worth noting that some people with Parkinson's disease experience movements that are too quick, not too slow. These unwanted accelerations are especially troublesome in speech and movement.
- **Slow movements:** also called "Bradykinesia", describes a general reduction of spontaneous movement, which can give the appearance of abnormal stillness.

It can be considered that elderly persons watch the television regularly, sometimes every day. During the time that elderly individuals are watching TV, they are often manipulating the remote control device of the television itself, or the remote control device of any video displaying system.

The proposed method and apparatus includes a generic remote control device that can replace any existing type of remote control by providing all the features for controlling the connected video system. The generic remote control device also includes several sensors that will be used to detect Parkinson's disease symptoms.

Thus, the proposed method and apparatus uses a combination of two sensors type to detect and analyze these symptoms:
- **Accelerometer:** An accelerometer is a compact device designed to measure non-gravitational acceleration. When the object that the accelerometer is integrated into goes from a standstill (no movement) to any velocity, the accelerometer is designed to respond to the vibrations associated with such movement. The accelerometer uses microscopic crystals that go under stress when vibrations occur, and from that stress a voltage is generated to create a reading on any acceleration. For purposes of the proposed method and apparatus, several accelerometers will be used to analyze the three standard axes (X, Y and Z axes). It should be noted that alternatively or additionally the accelerometers may be configured to analyze pitch, roll and yaw.
- **Gyroscope:** A gyroscope is a device that uses earth's gravity to help determine orientation. A gyroscope's design includes a freely-rotating disk called a rotor, mounted onto a spinning axis in the center of a larger and more stable wheel. As the axis turns, the rotor remains stationary to indicate the central gravitational pull, and, thus, which way is "down."

First, the generic remote control device of the proposed method and apparatus records every key manipulation. A goal is to identify different patterns occurring during the use of the remote control device. Here are a few examples (not exhaustive):
- Channel changing by typing every number: the time the monitored elderly individual takes between pressing/entering each digit can be recorded
- Volume changing: frequency of pressing on the same key for volume up or down can be recorded
- Etc...

The generic remote control device of the proposed method and apparatus also records the data coming from the sensors during this key manipulation. The proposed method and apparatus is able to link data coming from sensors to this pattern occurrence. By doing this, the proposed method analyzes the recorded data to identify an evolution in the elderly behavior when executing this pattern. Depending on this evolution, it will be possible to identify an evolution in the disease.

The generic remote control device of the proposed method and apparatus uses a Bluetooth connection to communicate with the collecting and analysis system. Bluetooth pairing needs to be established at the time of system installation. The collecting and analysis system needs to be connected to the Internet to send data to the collecting and analysis system. Possible strategies for connecting to the Internet include using the TV connectivity. Newer TVs already include the possibility of connecting to the Internet, and to support a Bluetooth connection. The ATSC 3.0 standard, in fact, mandates such connectivity. An application needs to be installed on the TV, and will run automatically as a silent service after installation. The role of the installed application is to establish the connection between the generic remote control device and a server. Alternatively, it is possible to use a home gateway. The home gateway may also have an installed application or the application may be installed on an additional device connected to the home gateway. Once again, the role of the installed application is to establish the connection between the generic remote control device and a server.

Collected data are stored in a database on the home gateway and sent (transmitted, forwarded) periodically to the server. Storage and analysis of data is performed on a server linked to the database. On the server, algorithms will add new data to the model, learn from it, and determine if a notification (alert) needs to be raised. It will also store the results of analysis for later consultation, to avoid unnecessary calculation time.

As an alternative of providing a new remote control device, existing sensors for recent SmartTVs can be used. For these kinds of devices (e.g., SmartTVs), included remote control devices frequently provide an accelerometers or gyroscopes that are mainly used for user navigation in the television user interface, or in games. Using the specific algorithms of the proposed method and apparatus enables the transformation of raw data from these sensors to analyzable data. The proposed method and apparatus still remains fully operational for detection and monitoring of Parkinson's disease symptoms. The remote control device of a SmartTV or equivalent smart device just needs to be connected to the collecting and analysis system of the proposed method and apparatus.

Sensor data will be combined to give precision to the detection and minimize false positive data. An algorithm needs to evaluate raw data from the remote control device to transform the raw data into usable data for analysis. Concretely, different actions need to be performed to evaluate data and determine if the data is useful to include the data in the analysis history, for example,
- Identify remote control key manipulation patterns
- Detect particular frequency on shaking. Compare with user's history and known patterns. Analyze evolution on shaking strength.
- Detect abnormal movement speed compared to usual. Analyze evolution of movement speed during time.

The proposed method and apparatus may be divided into two major phases: a training phase, where the behavior profile of the user is learned, and an anomaly detection phase, where the system tries to detect anomalies while the user is interacting with the device. Both phases include multiple steps.

The training phase includes data collection, data formatting, pattern mining, pattern selection, pattern characterization and anomaly detection learning. During the data collection step, the proposed method and apparatus collects data from two types of interactions of the user with the device (the generic or smart remote control device) including sensor data from the gyro-sensor and the accelerometer and user actions on the remote control device's buttons or on the screen (zoom, scroll, pattern, pressure). During the data formatting step, the proposed method and apparatus records user actions as a sequence of action items. During the pattern mining step, the proposed method and apparatus mines the action sequence to extract patterns of action items. During the pattern selection step, the proposed method and apparatus selects a subset of patterns (for example most frequent patterns). During the pattern characterization step, the proposed method and apparatus stores (records, logs) sensor data associated with each pattern in the selected subset of patterns, and for each occurrence of the pattern in the collected data. During the anomaly detection learning step, the proposed method and apparatus trains an anomaly detection classifier based on the subset of patterns and the associated sensor data.

The anomaly detection phase includes data collection, data formatting, pattern recognition, anomaly score computation and anomaly detection. During the data collection step, the proposed method and apparatus collects data from two types of interactions of the user with the device (the generic or smart remote control devices) including sensor data from the gyro-sensor and the accelerometer and user actions on the remote control device's buttons or on the screen (zoom, scroll, pattern, pressure). During the data formatting step, the proposed method and apparatus records user actions as a sequence of action items. During the pattern recognition step, the proposed method and apparatus mines the action sequence to extract occurrences of known patterns and their associated sensor data based on the previously selected subset of patterns, which were selected during the training phase. During the anomaly score computation step, the anomaly detection classifier of the proposed method and apparatus is executed for every pattern occurrence and associated sensor data, and outputs an anomaly score. During the anomaly detection step, the proposed method and apparatus raises an alert in the case of an anomaly (score above a threshold).

A detailed example of the proposed method and apparatus is as follows:
At each time period of the data collection and data mining of the training phase, the sensors send (transmit, forward) information (data) to a collection and analysis module. This information (data) is combined with the user's interactions with the generic or smart remote control device. In the following example, there are three samples of data. Each sample is described by a row vector of values. T_i represents the timestamp at time period i, Gj_i represents the gyro-sensor data j at step i, Aj_i represents the accelerometer data j at step i, and I_i is the action performed by the user (push button, zoom in, etc.) at step i.

| | | | | | | | |
|---|---|---|---|---|---|---|---|
| T_1 | G1_1 | G2_1 | G3_1 | A1_1 | A2_1 | A3_1 | I_1 |
| T_2 | G1_2 | G2_2 | G3_2 | A1_2 | A2_2 | A3_2 | I_2 |
| T_3 | G1_3 | G2_3 | G3_3 | A1_3 | A2_3 | A3_3 | I_3 |

The pattern mining step of the training phase of the proposed method and apparatus, mines the action sequence to extract patterns of action items: for example, the pattern [I_1 I_2 I_3] may appear several times in the data set, in which case the pattern is added to a list of action patterns. A list of action patterns is obtained, each described by an identifier P_i and its sequence of action items I_i:

| | | | |
|---|---|---|---|
| P1 | I_1 | I_2 | I_3 |

In the pattern selection step of the training phase, a pattern, for example, [I_1 I_2 I_3] is selected if the pattern belongs to the top N patterns occurring in the dataset. The threshold parameter N may have been previously defined by an administrator at start-up or configuration.

In the pattern characterization step of the training phase, for each pattern and each occurrence of the pattern in the collected data, the proposed method and apparatus stores (records, logs) the sensor data associated with the occurrence. In the example above, the proposed method and apparatus would store the following matrix as the sensor data associated with this occurrence of the pattern [I_1 I_2 I_3]:

| | | | | | | |
|---|---|---|---|---|---|---|
| T_1 | G1_1 | G2_1 | G3_1 | A1_1 | A2_1 | A3_1 |
| T_2 | G1_2 | G2_2 | G3_2 | A1_2 | A2_2 | A3_2 |
| T_3 | G1_3 | G2_3 | G3_3 | A1_3 | A2_3 | A3_3 |

In the anomaly detection learning step of the training phase, the proposed method and apparatus trains an anomaly detection classifier based on the subset of patterns and the associated sensor data. The training of the anomaly classifier may be by any one of the known methods for detecting anomalies in multivariate time series.

The training phase is invoked (executed) initially and may be invoked (executed) if a new individual is added to the environment and needs to be monitored or if new patterns are added or if the individual's condition or habits have evolved. That is, the training phase is not executed each and every time the anomaly detection phase is executed (invoked).

At each time period of the data collection and data mining steps of the anomaly detection phase, the sensors send (transmit, forward) information (data) to a collection and analysis module. This information (data) is combined with the user's interactions with the generic or smart remote control device. In the following example, there are three samples of data. Each sample is described by a row vector of values. T_i represents the timestamp at time period i, Gj_i represents the gyro-sensor data j at step i, Aj_i represents the accelerometer data j at step i, and I_i is the action performed by the user (push button, zoom in, etc.) at step i.

| | | | | | | | |
|---|---|---|---|---|---|---|---|
| T_1 | G1_1 | G2_1 | G3_1 | A1_1 | A2_1 | A3_1 | I_1 |
| T_2 | G1_2 | G2_2 | G3_2 | A1_2 | A2_2 | A3_2 | I_2 |
| T_3 | G1_3 | G2_3 | G3_3 | A1_3 | A2_3 | A3_3 | I_3 |

Given a new sequence of sensor data and user interactions, in the pattern recognition step of the anomaly detection phase, the proposed method and apparatus parses the sequence to find occurrences of known patterns from the list of previously selected patterns.

Once an occurrence of a known pattern is found, in the anomaly score computation step of the anomaly detection phase, the proposed method and apparatus tests the associated sensor data to determine if there is an anomaly. The classifier that was trained in the "Anomaly detection learning" step of the training phase is applied to the sensor data of the occurrence and the classifier outputs an anomaly score.

In the anomaly detection step, the proposed method and apparatus raises an alert in the case of an anomaly. For example, the anomaly score computed in the previous step may be above a threshold. The threshold parameter may have been previously defined by an administrator at start-up or configuration.

All the needed sensors are already included in smartphones or other smart devices. So it is possible to replace the generic or smart remote control device by the smartphone or other smart device. In this case, the smartphone or other smart device will need the installation of a dedicated application that will collect and analyze raw data and send alerts and monitoring information directly to a care giver or doctor. In this case the collected data types may include sensor data from the gyro-sensor and the accelerometer and user actions on the smart device such as zoom, scroll, pattern, pressure. So collected data will be different but exploitable by the proposed method and apparatus.

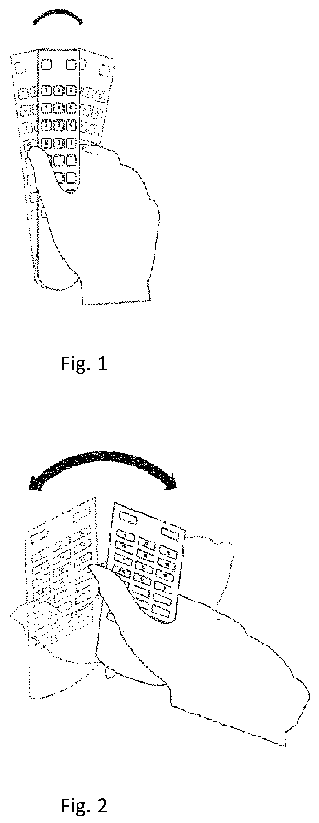

Fig. 1 is an illustration of a remote shaking movement (tremor symptom) of Parkinson's disease. The remote control device is shaking from left to right in the hand of an elderly person.

Fig. 2 is an illustration of a remote oscillating movement (tremor symptom) of Parkinson's disease. The remote control device oscillates or rolls in the elderly person's hand.

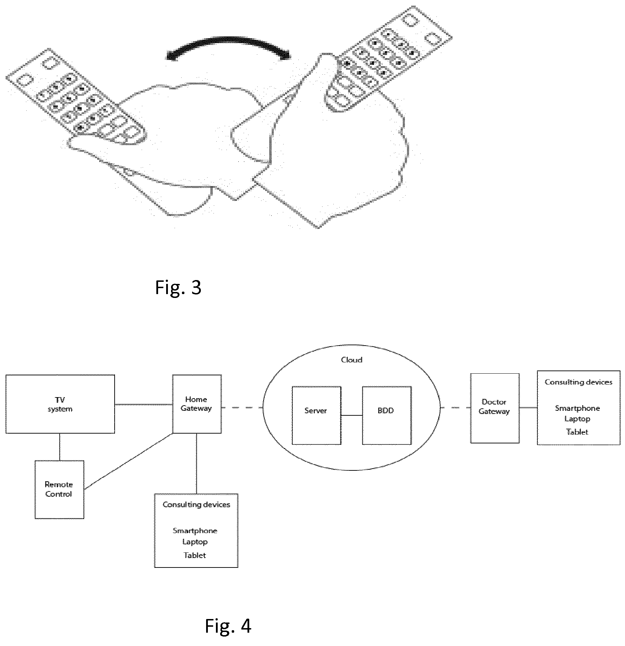

Fig. 3 is an illustration of a remote control device undergoing large movement (unwanted acceleration symptom) in the hand of an elderly person experiencing the symptoms of Parkinson's disease.

Fig. 4 is a schematic diagram of an exemplary embodiment of the proposed apparatus. The remote control device records the key strokes and gyro-sensor data as well as accelerometer data. The data is forwarded (sent, transmitted) to the home gateway. The data collected by the home gateway is forwarded (sent, transmitted) periodically to the server to be analyzed. That is, the home gateway may or may not be present or needed if the remote control device is in communication with the server. Additionally or alternatively, other devices such as smartphones, laptops, tablets etc. may collect data. Data from these smart devices may also be forwarded (sent, transmitted) to the home gateway for periodic transmission to the server or the smart device may be in direct communication with the server. At the other end the doctor gateway may be in communication with the server and also in communication with smart devices at the doctor's office.

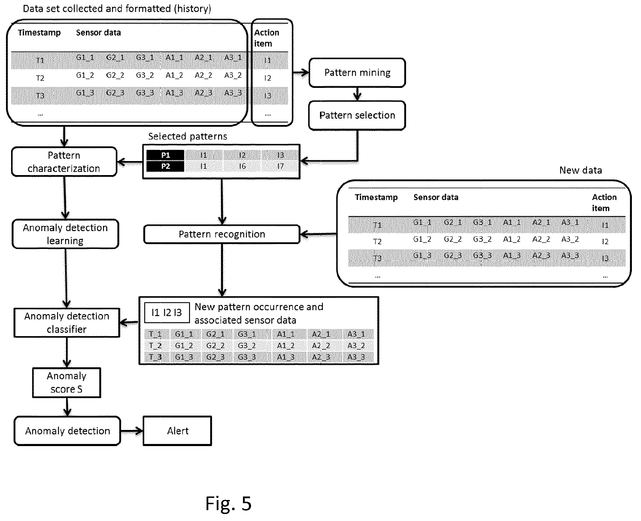

Fig. 5 is an exemplary embodiment of the proposed method and apparatus. At the top left the data are collected and formatted in the data collection and data formatting steps of the training phase. That is, the data are forwarded to and accepted by the server from either the home gateway, which may be used to temporarily store the data, or directly from the remote control devices (generic or smart). The data are collected for an initial (first) period of time. Specifically, during the data collection step, the proposed method and apparatus collects data from two types of interactions of the user with the device (the generic or smart remote control device) including sensor data from the gyro-sensor and the accelerometer and user actions on the remote control device's buttons or on the screen (zoom, scroll, pattern, pressure). During the data formatting step, the proposed method and apparatus records user actions as a sequence of action items. During the pattern mining step, the proposed method and apparatus mines the action sequence to extract patterns of action items. During the pattern selection step, the proposed method and apparatus selects a subset of patterns (for example most frequent patterns). During the pattern characterization step, the proposed method and apparatus stores (records, logs) sensor data associated with each pattern in the selected subset of patterns, and for each occurrence of the pattern in the collected data. During the anomaly detection learning step, the proposed method and apparatus trains an anomaly detection classifier based on the subset of patterns and the associated sensor data.

The new data at the right have already been collected and formatted for a subsequent (second) period of time as part of the anomaly detection phase. That is, the data are forwarded to and accepted by the server from either the home gateway, which may be used to temporarily store the data, or directly from the remote control devices (generic or smart). During the data collection step of the anomaly detection phase, the proposed method and apparatus collects data from two types of interactions of the user with the device (the generic or smart remote control devices) including sensor data from the gyro-sensor and the accelerometer and user actions on the remote control device's buttons or on the screen (zoom, scroll, pattern, pressure). During the data formatting step of the anomaly detection phase, the proposed method and apparatus records user actions as a sequence of action items. During the pattern recognition step, the proposed method and apparatus mines the action sequence to extract occurrences of known patterns and their associated sensor data based on the previously selected subset of patterns. During the anomaly score computation step, the anomaly detection classifier of the proposed method and apparatus is executed for every pattern occurrence and associated sensor data, and outputs an anomaly score. During the anomaly detection step, the proposed method and apparatus raises an alert in the case of an anomaly (score above a threshold).

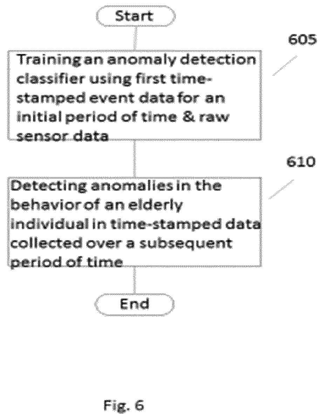

Fig. 6 is a flowchart of an exemplary embodiment of the proposed method and apparatus. The proposed method is executed in (performed by) a server other than the data collection module by the remote control devices (generic or smart). That is, the data are forwarded to and accepted by the server from either the home gateway, which may be used to temporarily store the data, or directly from the remote control devices (generic or smart). It should be noted that the server may be within or co-located with a smart remote control device or the home gateway. At 605 the anomaly detection classifier is trained using first time-stamped event data for an initial (first) period of time and raw sensor data. The training phase (605) is invoked (executed) initially and may be invoked (executed) if a new individual is added to the environment and needs to be monitored or if new patterns are added or if the individual's condition or habits have evolved. At 610 anomalies are detected in the behavior of an elderly monitored individual using time-stamped event data collected over a subsequent period of time. The anomaly detection classifier is used to determine if the second time-stamped event data (data for the anomaly detection phase) for a second (subsequent) period of time and raw sensor data constitute an anomaly.

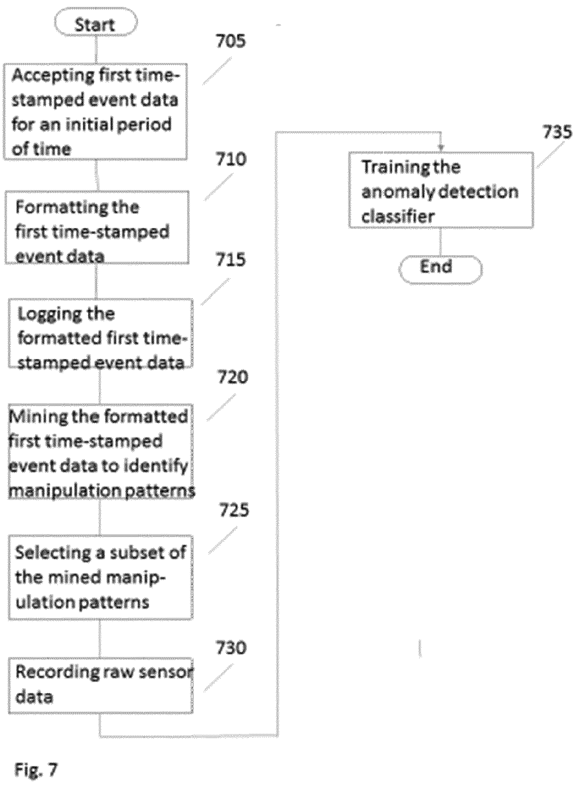

Fig. 7 is a flowchart of an exemplary embodiment of the training phase of the proposed method and apparatus. At 705 time-stamped event data of initial behavior is accepted from sensors for the initial period of time. At 710 the first time-stamped data of the initial behavior of the monitored individual over the initial period of time may be formatted if necessary. The formatting of the data includes preprocessing aimed at making the collected data cleaner, more homogeneous, with less errors, and easy to feed to the other modules. However this step is not compulsory, for example, as in the case where the collected data already has these qualities from the start. Also, this step may be included in other modules such as the data collection module, or the logging module, or the mining modules. At 715 the formatted first time-stamped data is logged. At 720 the logged formatted first time-stamped event data is mined to identify mined manipulation patterns of a device having sensors. At 725 a subset of the mined manipulation patterns is selected based on frequency of occurrence of the manipulation patterns and stored in a database shared with the anomaly detection phase. At 730 the raw sensor data is recorded for each occurrence of each selected manipulation pattern. At 735 the anomaly detection classifier is trained and the results and the classifier are stored in a database shared with the anomaly detection phase.

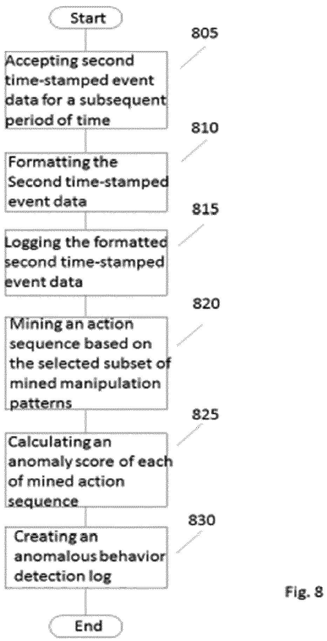

Fig. 8 is a flowchart of an exemplary embodiment of the anomaly detection phase of the proposed method and apparatus. At 805 second time-stamped event data of subsequent behavior from sensors in the environment for a subsequent period of time is accepted. At 810 the second time-stamped event data of subsequent behavior of the monitored individual may be formatted if necessary. The formatting of the data includes preprocessing aimed at making the collected data cleaner, more homogeneous, with less errors, and easy to feed to the other modules. However this step is not compulsory, for example, as in the case where the collected data already has these qualities from the start. Also, this step may be included in other modules such as the data collection module, or the logging module, or the mining modules. At 815 the formatted second time-stamped event data of subsequent behavior of the monitored individual is logged. At 820 an action sequence based on the selected subset of mined manipulation patterns is mined. At 825 an anomaly score of each mined action sequence is calculated using the anomaly detection classifier. At 830 an anomalous behavior detection log is created based on the anomaly scores.

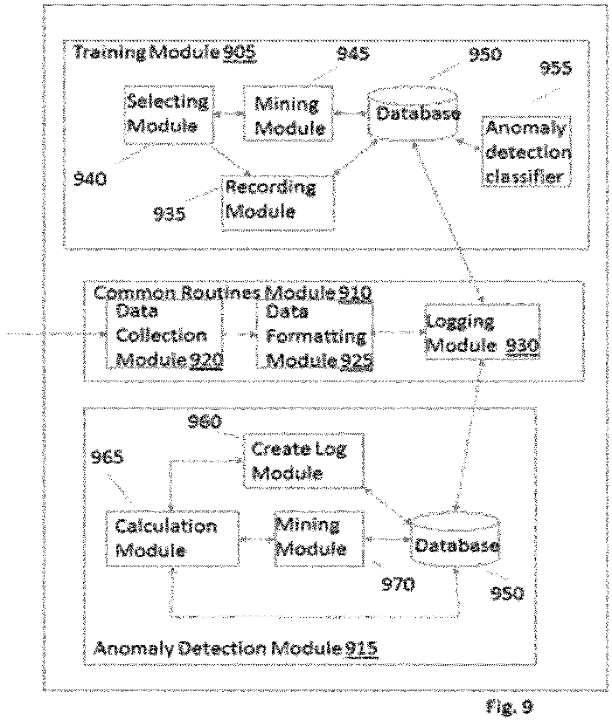

Fig. 9 is a block diagram of an exemplary embodiment of the proposed method and apparatus. The proposed method is executed in (performed by) a server than the data collection module by the remote control devices (generic or smart). That is, the data are forwarded to and accepted by the server from either the home gateway, which may be used to temporarily store the data, or directly from the remote control devices (generic or smart). It should be noted that the server may be within or co-located with a smart remote control device or the home gateway. The server also includes a communication module (not shown) to communicate with the remote control devices (generic or smart) or the home gateway in order to receive the data regarding the monitored individual's interactions (key strokes) with the remote control devices (generic or smart) and sensor data from the gyroscope and accelerometer. The communication module also supports communication of the analyzed results to a care giver. The care giver may be a family member or medical staff or non-medical care staff. The proposed method and apparatus includes a training module 905, an anomaly detection module 915 and a common routines module 910. The common routines module includes a data collection module 920, a data formatting module 925 and logging module 930. These three routines are considered common routines for the purposes of discussion because they are used for both first time-stamped event data during an initial period of time and second time-stamped event data during a subsequent period of time. These routines could just as easily be included in both of the training module and the anomaly detection module and not be included in a common routine module. The data is collected by the data collection module 920 and formatted by the data formatting module 925. The formatted data is forwarded to a logging module 930, which logs (records, stores) the data in a database 950. The data is recorded (stored, logged) in any form of storage device including magnetic disks, optical disks, RAM, etc. The mining module 945 of the training module mines the formatted first time-stamped event data to identify manipulations patterns of a device having sensors. The selecting module 940 of the training module selects a subset of mined manipulation patterns based on frequency of occurrence of the manipulation patterns. Recording module 935 of the training module records (stores, logs) the link between the selected manipulation patterns and the formatted data, which are both already recorded (stored, logged) in database 950. The anomaly detection classifier 955 is trained and the anomaly detection classifier and training results are stored in database 950, which is accessible by both the training module and the anomaly detection classifier. The results of the training phase are parameters of a model/classifier which need to be saved in a database and shared with the anomaly detection module.

The formatted second time-stamped event data is forwarded to logging module 930 of the anomaly detection module 915, which logs (records, stores) the data in a database 950. Please note that database 950 is accessible to both the training module and the anomaly detection module and may reside in either the training module or the anomaly detection module. The data is recorded (stored, logged) in any form of storage device including magnetic disks, optical disks, RAM, etc. The mining module 970 of the anomaly detection module 915 mines an action sequence based on the selected subset of mined manipulation patterns. Calculation module 965 calculates an anomaly score of each mined action sequence. The create log module 960 creates an anomalous behavior detection log based on the anomalous scores.

It is to be understood that the proposed method and apparatus may be implemented in various forms of hardware, software, firmware, special purpose processors, or a combination thereof. Special purpose processors may include application specific integrated circuits (ASICs), reduced instruction set computers (RISCs) and/or field programmable gate arrays (FPGAs). Preferably, the proposed method and apparatus is implemented as a combination of hardware and software. Moreover, the software is preferably implemented as an application program tangibly embodied on a program storage device. The application program may be uploaded to, and executed by, a machine comprising any suitable architecture. Preferably, the machine is implemented on a computer platform having hardware such as one or more central processing units (CPU), a random access memory (RAM), and input/output (I/O) interface(s). The computer platform also includes an operating system and microinstruction code. The various processes and functions described herein may either be part of the microinstruction code or part of the application program (or a combination thereof), which is executed via the operating system. In addition, various other peripheral devices may be connected to the computer platform such as an additional data storage device and a printing device.

It should be understood that the elements shown in the figures may be implemented in various forms of hardware, software or combinations thereof. Preferably, these elements are implemented in a combination of hardware and software on one or more appropriately programmed general-purpose devices, which may include a processor, memory and input/output interfaces. Herein, the phrase "coupled" is defined to mean directly connected to or indirectly connected with through one or more intermediate components. Such intermediate components may include both hardware and software based components.

It is to be further understood that, because some of the constituent system components and method steps depicted in the accompanying figures are preferably implemented in software, the actual connections between the system components (or the process steps) may differ depending upon the manner in which the proposed method and apparatus is programmed. Given the teachings herein, one of ordinary skill in the related art will be able to contemplate these and similar implementations or configurations of the proposed method and apparatus.

## Claims

1. A method of creating an anomalous behavior detection log for reporting anomalous behavior of a monitored individual in an environment, said method comprising:
training (735) an anomaly detection classifier using first time-stamped event data for an initial period of time and raw sensor data;
accepting (805) second time-stamped event data of subsequent behavior from sensors in said environment for a subsequent period of time;
logging (815) said second time-stamped event data of subsequent behavior of the monitored individual;
mining (820) an action sequence based on a selected subset of mined manipulation patterns;
calculating (825) an anomaly score of each mined action sequence using the anomaly detection classifier; and
creating (830) an anomalous behavior detection log based on said anomaly scores.

2. The method according to claim 1, further comprising:
accepting (705) first time-stamped event data of initial behavior from sensors in said environment for said initial period of time;
logging (715) said first time-stamped event data of said initial behavior of said monitored individual over said initial period of time in the environment;
mining (720) said logged first time-stamped event data to identify said mined manipulation patterns of a device having sensors;
selecting (725) said subset of said mined manipulation patterns based on frequency of occurrence of said manipulation patterns; and
recording (730) information regarding a link between said selected manipulation patterns and said first time-stamped event data.

3. The method according to claim 1, wherein said second time-stamped event data includes the monitored individual's interactions with a remote control device and sensor data from a gyroscope and an accelerometer.

4. The method according to claim 3, wherein said accelerometer data is X, Y and Z axis data or pitch, roll and yaw data.

5. The method according to claim 1, wherein anomalous behavior is communicated to a care giver.

6. The method according to claim 2, wherein said first time-stamped event data includes the monitored individual's interactions with a remote control device and sensor data from a gyroscope and an accelerometer.

7. The method according to claim 6, wherein said accelerometer data is X, Y and Z axis data or pitch, roll and yaw data.

8. A server for creating an anomalous behavior detection log for reporting anomalous behavior of a monitored individual in an environment, said method comprising:
means for training an anomaly detection classifier (955) using first time-stamped event data for an initial period of time and raw sensor data;
means for accepting second time-stamped event data (920) of subsequent behavior from sensors in said environment for a subsequent period of time;
means for logging said second time-stamped event data (930) of subsequent behavior of the monitored individual;
means for mining (970) an action sequence based on a selected subset of mined manipulation patterns;
means for calculating an anomaly score (965) of each mined action sequence using the anomaly detection classifier; and
means for creating an anomalous behavior detection log (960) based on said anomaly scores.

9. The server according to claim 8, further comprising:
means for accepting first time-stamped event data (920) of initial behavior from sensors in said environment for said initial period of time;
means for logging said first time-stamped event data (930) of said initial behavior of said monitored individual over said initial period of time in the environment;
means for mining said first time-stamped event data (945) to identify said mined manipulation patterns of a device having sensors;
means for selecting said subset of said mined manipulation patterns (940) based on frequency of occurrence of said manipulation patterns; and
means for recording information (935) regarding a link between said selected manipulation patterns and said first time-stamped event data.

10. The server according to claim 8, wherein said second time-stamped event data includes the monitored individual's interactions with a remote control device or sensor data from a gyroscope and an accelerometer.

11. The server according to claim 10, wherein said accelerometer data is X, Y and Z axis data or pitch, roll and yaw data.

12. The server according to claim 8, wherein anomalous behavior is communicated to a care giver.

13. The server according to claim 9, wherein said first time-stamped event data includes the monitored individual's interactions with a remote control device or sensor data from a gyroscope and an accelerometer.

14. The server according to claim 13, wherein said accelerometer data is X, Y and Z axis data or pitch, roll and yaw data.
